# EUROPEAN PATENT APPLICATION

(11) **EP 1 070 733 A1**
(43) Date of publication of application: **24.01.2001**
(21) Application number: 99114308.2
(22) Date of filing: 21.07.1999
(51) Int. Cl.: C08G 59/14, C08G 59/18

(54) **Method of synthesis of polyaminofunctional hydroxyurethane oligomers and hybride polymers formed therefrom**

(71) Applicant: Polymate Ltd., Haifa 34752 (IL); Chemonol Ltd., Nesher 36601 (IL)
(72) Inventor: Figovsky, Oleg, Haifa 34750 (IL); Shapovalov, Leonid, Haifa 35437 (IL); Blank, Nelly, Haifa 34750 (IL); Buslov, Florida, Kyriat Ata 28000 (IL)
(74) Representative: Graf, Helmut, Dipl.-Ing.

(57) **Abstract**

Chemically resistant materials with high mechanical properties are provided by using adducts of primary diamines (with oligocyclocarbonate and epoxy compounds) and epoxy oligomers with ended epoxy groups (or mixture of epoxy oligomers and oligomercaptanes).

## Description

### Technical Field

The present invention is related to epoxyurethane oligomers without isocyanates and polymer compounds based on them and, more specifically, to coatings, adhesives, composite materials, sealants, synthetic leather and to methods of producing such materials.

### Background of the Invention

It is known method of preparing polymers by using aminourethane oligomers (without requiring the use of isocyanates) and an epoxide resin to form hardener crosslinked polymer (USA patent 5175231_{,} published Dec 29, 1992).
But it is impossible to prepare nonisocyanate polyurethane-epoxyde materials using mixture containing both epoxy and cyclocarbonate groups because of simultaneously reactions of epoxy and cyclocarbonate groups with the primary amino groups. So far as cyclocarbonate groups react only with primary aminogroups many cyclocarbonate groups do not react. So in such mixture after hardening cyclocarbonate compounds only plasticize the epoxy polymer and it is impossible to prepare epoxy-polyurethane materials with high properties.

### Summary of the Invention

One embodiment of the present invention relates to oligomers containing epoxy and cyclocarbonate groups, adduct of aliphatic or cycloaliphatic diamines with these oligomers. Such oligomers may be used for curing epoxy resins, for preparing constructive glues, sealants, coatings, construction, etc.

### Detailed Description of the Invention

Polyfunctional oligomers of the present invention are based upon oligomers with epoxy and cyclocarbonate groups, which was prepared by the reaction of carbon dioxide and an epoxy oligomer with catalizer according our pat. (Patent Israel 122763).

Such oligomers containing epoxy and cyclocarbonate groups react with aliphatic or cycloaliphatic amines by epoxy and cyclocarbonate groups. These adducts are used as hardeners for epoxy oligomers or mixture of epoxy oligomers and oligomercaptanes.

For preparing epoxy-polycyclocarbonate adduct are used epoxy-cyclocarbonate olygomers preferably of such formula:

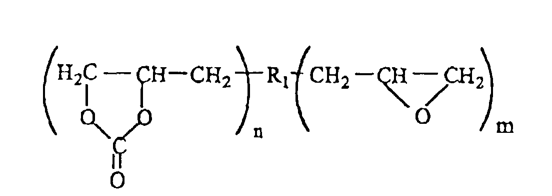

Suitable commercially available epoxy resins

| | |
|---|---|
| DER-324, DER-332, DEN-431 | Dow Chemical Co (USA), |
| EPON 813, EPON 8021, EPON 8091, | |
| EPON 825, EPON 828 | Shell Chemical Co (USA), |
| NPEF-165 | Nan Ya Plastic Corporation (R. China), |
| PEP 6180, PEP 6769, PEP 6760 | Pacific Epoxy Polymers, Inc(USA), |
| Araldite 0510, MY-0501, MY-720 | Ciba-Geigy A.G. (USA), |
| Laprolat 803, 703 | "Macromer" Ltd. (Russia), |
| Ricotuff 1000-A, Ricotuff 1100-A, | |
| Ricotuff 1110-A, Ricopoxy 30 | Ricon Resins, Inc. (USA), |
| Setalux AA-8502, 8503 | AKZO NOBEL (Netherland), |
| Eponex 1510,1511 | Shell Chemical Co. (USA). |

Preferably commercially available primary amines: N,N-bis(3-aminopropyl) methylamine (BASF, Germany), m-xylylenediamine (Mitsubishi, Japan), triethyleneglycoldiamine (Jeffamine), EDR-148 (Huntsman Corporation, USA), 1,3-diaminopentane (Du Pont, USA), Isoforodiamine (BASF, Germany), Jeffamine T-403 (Huntsman Corporation, USA), etc.

### Examples

The following examples of polyaminofunctional urethane oligomers and hybride polymers formed therefrom. However, it is to be understood that the examples are for illustrative purposes only and in no manner is the present invention limited to the specific disclosure therein.

### Example 1: Preparing Polyaminofunctional urethane Oligomers

Ten sample compositions of the invention were prepared by the following method. The components formulated into each sample and the amounts used are shown in Table 1.

Synthesis is a reaction between epoxy and cyclocarbonate groups with polyamines. Such process is working out in special glass reactor under N₂. Contents are mixed during 10 min at the 20°C, after that the reaction continues during 0,5-1 hr by 80-120°C (or 24 hrs by 20-25°C). After this operation according with Table 1 oligomers are mixed with epoxy resins for curing (according Table 1) with adding fillers and pigments or without.

After that the reaction of curing continues during 2 hrs by the 110-120°C or 7 days at 20°C.

For preparing coatings were used 80% solutions in butylglycol or dimethylformamide.

Additionally, certain control samples were also prepared by the above method.

**Composition 1c** is identical to Composition 1 except that epoxy-cyclocarbonate oligomer with EEW = 331; CC EW = 615.4 was used in the reaction of oligomerisation.

**Composition 2c** is identical to Composition 1 except that epoxy-cyclocarbonate olygomer with EEW = 653; CC EW = 352 was used in the reaction of oligomerisation.

**Composition 3c** is identical to Composition 3 except that epoxy-cyclocarbonate olygomer with EEW = 248; CC EW = 744 was used in the reaction of oligomerisation.

**Composition 4c** is identical to Composition 4 except that epoxy-cyclocarbonate oligomer with EEW = 2000; CC EW = 240 was used in the reaction of oligomerisation.

**Composition 5c** is identical to Composition 5 except that epoxy-cyclocarbonate oligomer with EEW = 921; CC EW = 196 was used in the reaction of oligomerisation.

**Composition 6c** is identical to Composition 6 except that epoxy-cyclocarbonate oligomer with EEW = 161; CC EW = 758 was used in the reaction of oligomerisation.

**Composition 7c** is identical to Composite 7 except that epoxy-cyclocarbonate oligomer with EEW = 193 ; CC EW = 358 was used in the reaction of oligomerisation.

**Composition 8c** is identical to Composite 8 except that epoxy-cyclocarbonate oligomer with EEW = 396; CC EW = 213 was used in the reaction of oligomerisation.

**Composition 9c** is identical to Composite 9 except that epoxy-cyclocarbonate oligomer with EEW = 426; CC EW = 1401 was used in the reaction of oligomerisation.

**Composition 10c** is identical to Composite 10 except that epoxy-cyclocarbonate oligomer with EEW = 1502; CC EW = 457 was used in the reaction of oligomerisation.

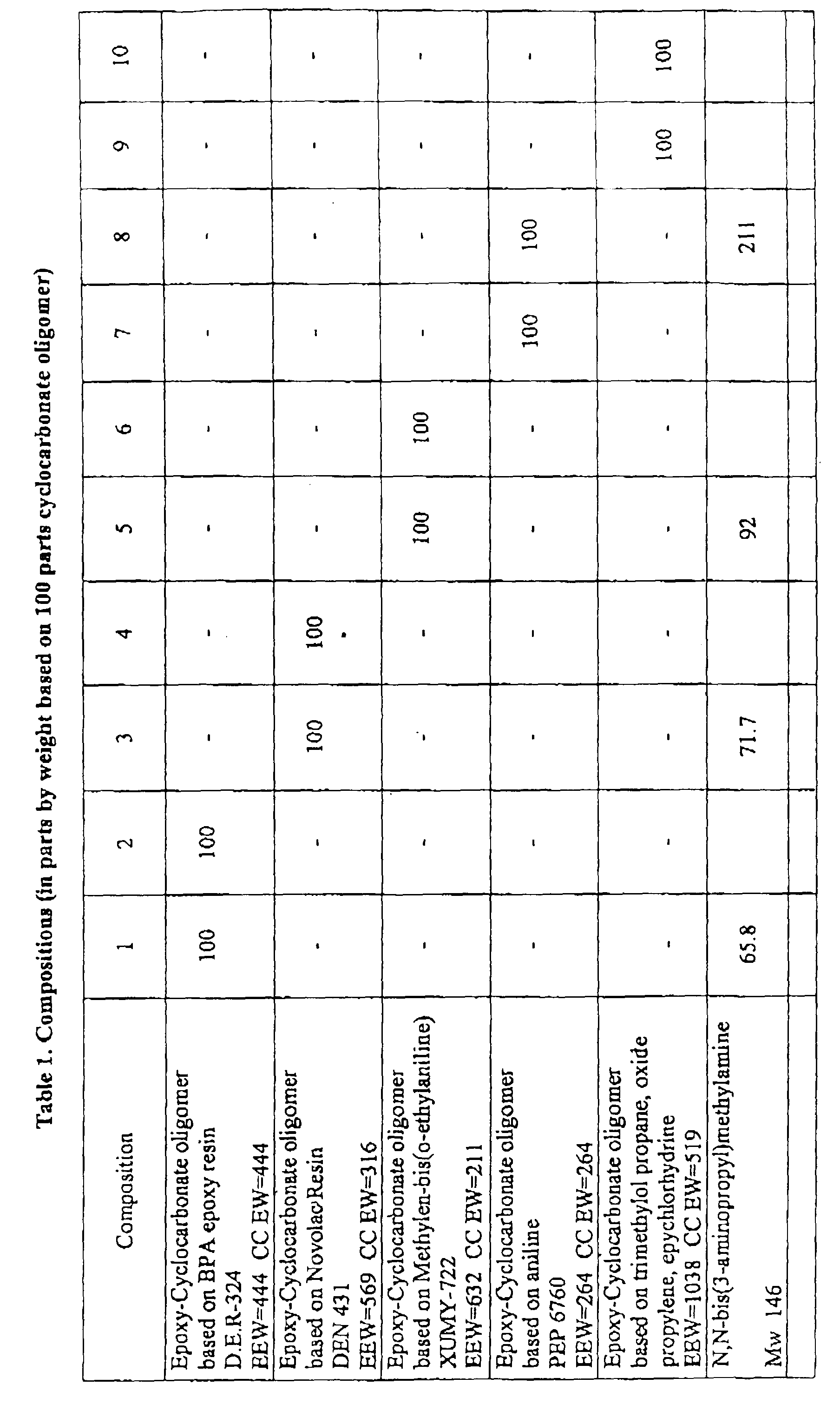

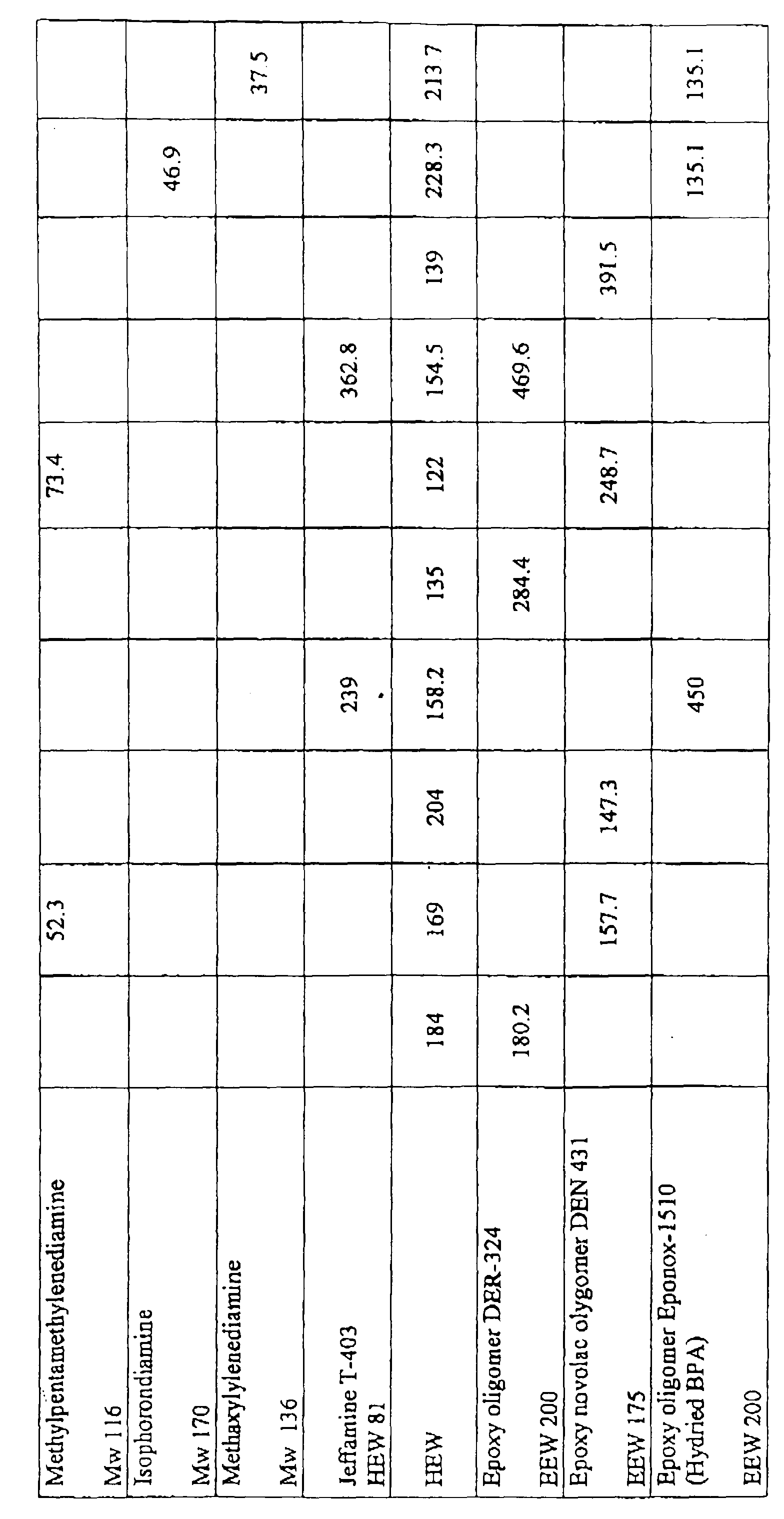

### Examples 2: Preparing NIPU hybrid coatings

Cyclocarbonate oligomers (1-10) prepared according the method described in Example 1 were used to coat a cleaned steel plate. Each composition formed a coating layer with a thickness of 1-2 mm. Then the coating was hardened at a temperature 110°C for 2 hours. These coated steel samples were used to determine the adhesion of each coating to the steel substrate according to the method prescribed by ASTM D3359-93. Compositions were used as 80% solution in butylglycol. For the purposes of tensile testing, the above procedure was modified by using a polytetrafluoroethylene (PTFE) sheet instead of steel plate, so a free film of each of the 10 cyclocarbonate-oligomers could be obtained by peeling the PTFE from the cured film. The tensile properties, i.e. tensile strength and elongation at break, of each free cured film were determined according to the method prescribed by ASTM D638-84. Such samples were also used to determine the coefficient of chemical resistance of each coating by the procedure discussed above in which the tensile strength was used to determine K_{CR}.

The properties of ten coatings of the invention, samples 11-20, are shown in Table 2.

The properties of ten coatings, samples 21-30, are also formed by the above described method are shown in Table 3.

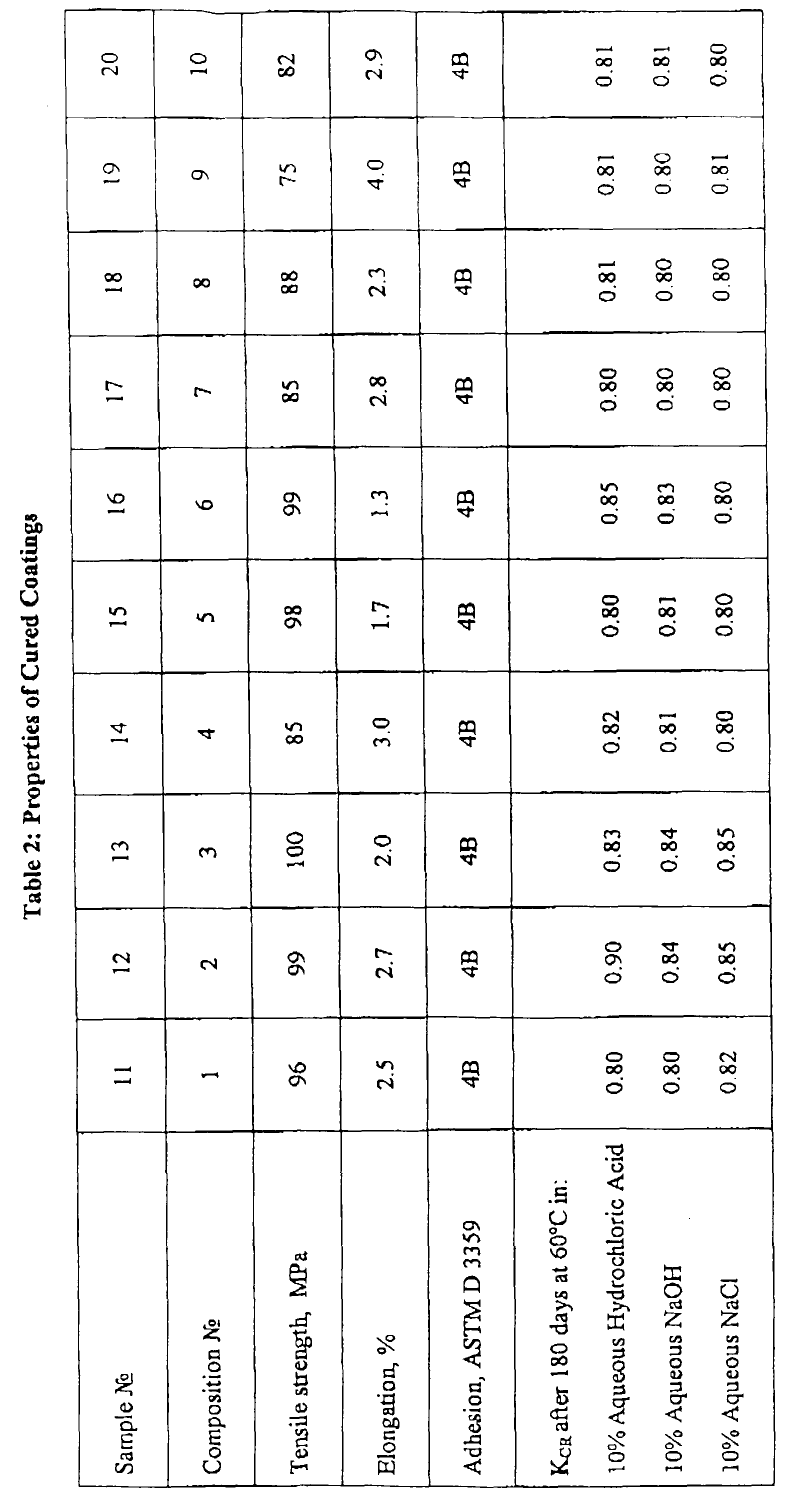

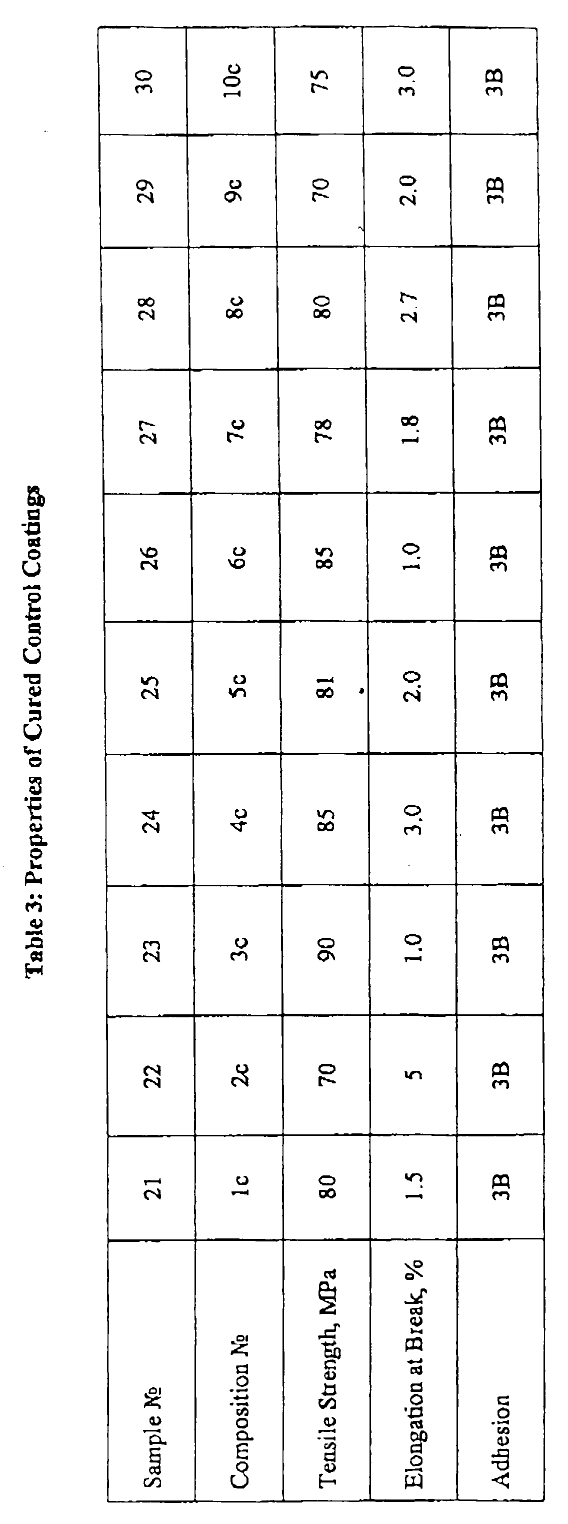

The proposed oligomers may be used for preparing adhesives, sealants, synthetic foams, synthetic leathers, hardeners for epoxy resins, casting elastomers, structure plastics, etc.

The possible areas of applications are illustrated by following examples. Adhesives based on the compositions 1-10 (table 1) can be used as structure adhesives with high service properties. The adhesives compositions have been received by adding to adducts (1-10) epoxy resins, which are using here as a hardener. Such method of Adhesives preparing gives adhesives joints with more shock and vibration resistance properties. The mechanical properties of adhesives joints were described in the table 2.

By preparing of adhesives compositions pigments and fillers can be also added. Preferable ones are barium sulfate, titanium dioxide, silica and ferrous oxides pigments and aluminate cement. By adding as a filler glass microspheres can be received synthetic foams.

Sealants can be received on the base of adducts by example 1, 3, 7. In this case are needed using another hardeners (see table 4). The properties of such sealants are given in the table 5.

On the base of oligomers according to example 1-10 can be also prepared reinforced plastics with glass, carbon or kevlar fibers and polymerconcretes with high impact, shock and abrasive resistance, with 2-3 times higher mechanical properties than those of well known unsaturated polyester reinforced plastics and concretes.

For the purposes of fast curing it is possible to use usual accelerators. The above procedure was modified by using an olygomeric mercaptane (polysulfides).

Four compositions of the invention were prepared by adding oligomercaptane (Capcure 3-800) and epoxy olygomer to the compositions 1.3.5.8 (according table 1) with adding additional amounts of epoxy olygomers.

Reference is made to table 6 and samples 31-34.

The curing time of four coatings (samples 31-34) in comparison with analogous samples without accelerator is shown in table 7.

**Table 4:**

| **Compositions for Sealants (parts by weight)**. | | | | | | |
|---|---|---|---|---|---|---|
| Sample Nº | 21 | 22 | 23 | 24 | 25 | 26 |
| Composition | 11 | 12 | 13 | 14 | 15 | 16 |
| Epoxy Cyclocarbonate oligomer based on BPA epoxy resin DER-324 EEW=444 CCEW=444 | 100 | - | - | 100 | - | - |
| Epoxy Cyclocarbonate oligomer based on Novolac Resin DEN 431 EEW=569 CCEW=316 | - | 100 | - | - | 100 | - |
| Epoxy Cyclocarbonate oligomer based on aniline PER 6760 EEW=264 CCEW=264 | - | - | 100 | - | - | 100 |
| N,N bis(3 aminopropyl) methylamine Mw 146 | 65.8 | 71.9 | - | 65.8 | 71.9 | - |
| m-Xylelenediamine Mw 136 | - | - | 103 | - | - | 103 |
| Molecular weight of adduct | 736 | 978 | 539 | 736 | 978 | 536 |
| HEW | 147.2 | 148 | 107 | - | - | - |
| Epoxy oligomer D.E.R-732 EEW=320 | 361 (78%) | 372 (79%) | 607 (86%) | - | - | - |
| Epoxy oligomer Ricopoxy 30 EEW=717 | - | - | - | 809 89% | 833 89% | 1360 93% |

**Table 5:**

| **Sealants properties.** | | | | | | |
|---|---|---|---|---|---|---|
| Sample Nº | 21 | 22 | 23 | 24 | 25 | 26 |
| Composition | 11 | 12 | 13 | 14 | 15 | 16 |
| Tensil strength, MPa | 60 | 58 | 56 | 46 | 42 | 42 |
| Elongation at Break, % | 100 | 110 | 115 | 360 | 400 | 500 |

**Table 6.**

| **Composition** | *31* | *32* | *33* | *34* |
|---|---|---|---|---|
| *Epoxy-Cyclocarbonate olygomer on BPA epoxy resins DER-324 EEW-444, CCEW-444* | *100* | - | - | - |
| *Epoxy-Cyclocarbonate olygomer based on Novolac resin DEN-431 EEW-569, CCEW-316* | - | *100* | - | - |
| *Epoxy-Cyclocarbonate olygomer based on Methylen-bis(o-eyhtlamine)* | - | - | *100* | - |
| *Epoxy-Cyclocarbonate olygomer based on aniline PEP 6760 EEW-264, CCEW-264* | - | - | - | *100* |
| *N,N-bis(3-aminopropyl)methyl amine* | *65.8* | *71.7* | *92* | *211* |
| *HEW* | *184* | *204* | *135* | *139* |
| *Olygomercaptane Capcure 3-800 EW-270* | *135* | *135* | *135* | *135* |
| *Epoxy olygomer DER-324, EEW-200* | *280* | *247* | *384* | *491* |

**Table 7.**

| **Curing time of coatings.** | | | | | | | | |
|---|---|---|---|---|---|---|---|---|
| **Sample No** | | | | | | | | |
| | **1** | **31** | **3** | **32** | **5** | **33** | **8** | **34** |
| *Curing time, h* | *18* | *2* | *18* | *3* | *18* | *4* | *18* | *4* |

## Claims

1. Polyaminofunctional oligomers 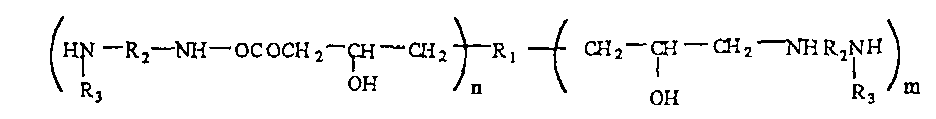 where:
R₁ ― aliphatic, cycloaliphatic, oligoester or oligoether radicals
R₂, R₃ ― H, alkyl, aryl alkylaryl;
m = 0.1-2; n= 1-5

2. The method of preparing of polyaminofunctional oligomers by reaction of compounds 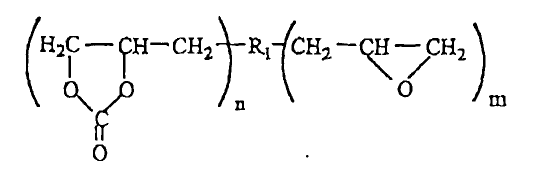 with primary diamines by stoichiometric ratio to epoxy and cyclocarbonate groups.

3. The method according p.2, includes reaction carried out by temperature from 80°C to 120°C both in "situ" and in organic solvents.

4. The method according claim 2 or 3 includes using oligomers, chosen from the group which includes oligomers having the following structures: 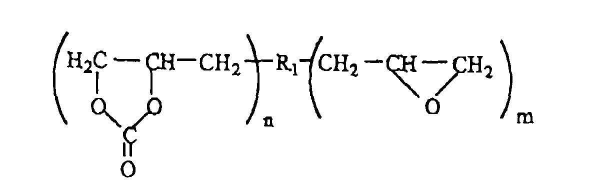 where R₁ = 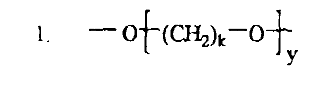 k = 2 - 6, y = 1 - 20, n = 1 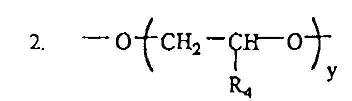 where: R₄=CH₃ , C₂H₅ y = 1 - 20 , n = 1 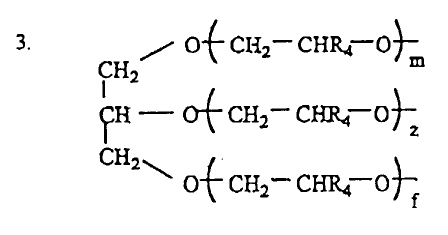 where: R₄ = H, CH₃ m + z + f = 0 - 50 n = 2 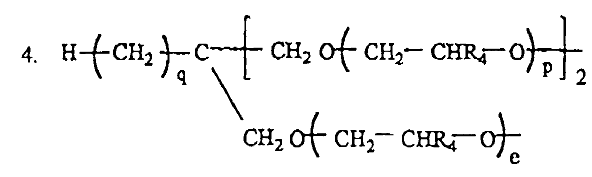 where: R₄ = H, CH₃ p + e = 0 - 50 q = 1 - 2 n = 2 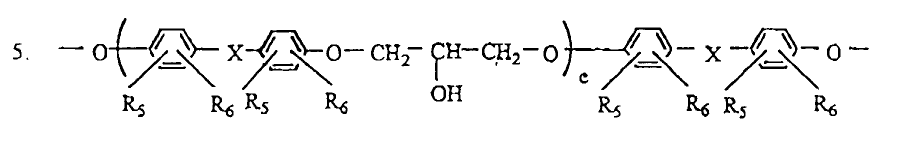 where: X = ―CH₂― , (CH₃)₂C〈 , ―SO₂― , (CF₃)₂C〈 ; R₅, R₆ = ―H, ―CH₃, ―Cl, ―Br; e = 0 - 5 , n = 1 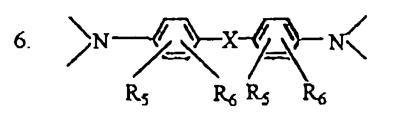 where: X = ―CH₂―, (CH₃)₂C〈, ―SO₂― , 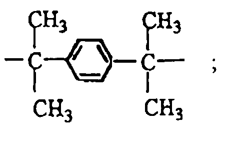 R₅, R₆ = ―H, ―CH₃, ―Cl; n = 3 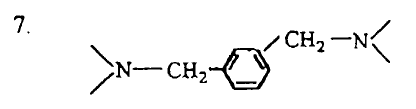 n=3 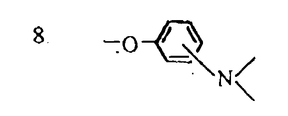 n=2 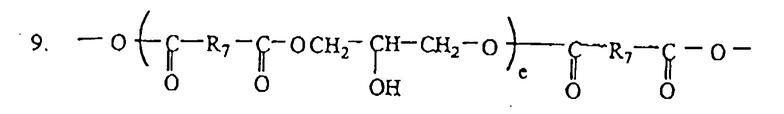 where: R₇ = 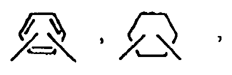 ―(CH₂)ₘ― ; e = 0 - 2, n = 1 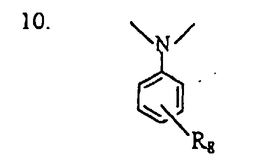 where: R₈ = ―H , ―CH₃, ―Br , ―Cl; n = 1 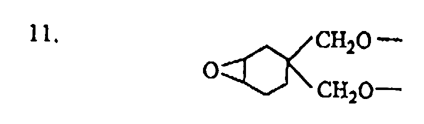 n = 1 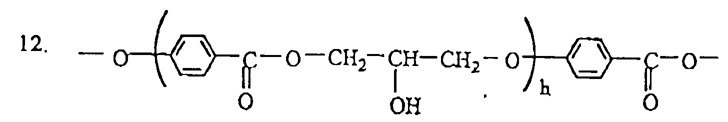 h = 0 - 2 n = 1 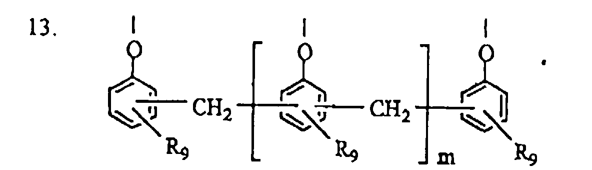 where: R₉ = ―H, ―CH₃ ; m = 1 - 5, n = 2 - 6 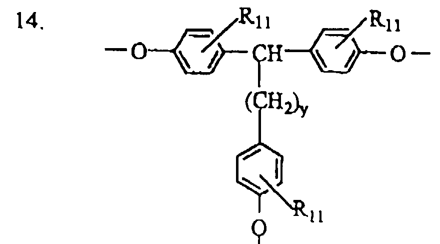 where: R₁₁ = ―H , ―CH₃ ; y = 1 - 2, n=2 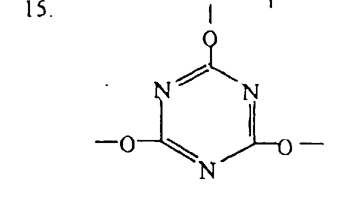 n = 1 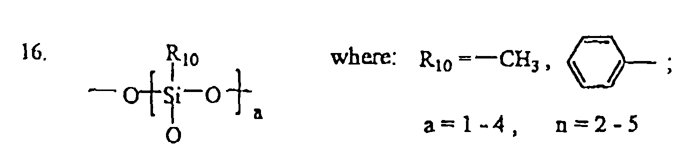 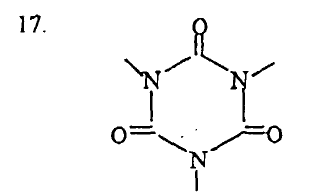 n = 2 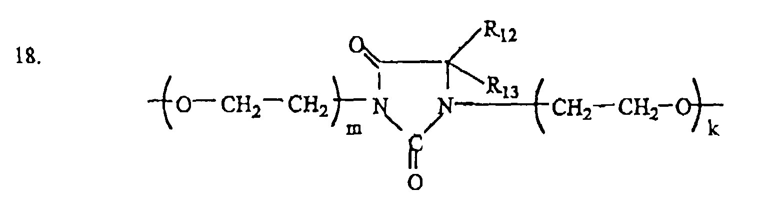 where: R₁₂, R₁₃ = H, CH₃; m + k = 0 - 6, n = 1 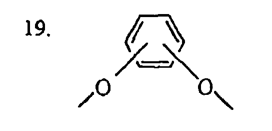 n = 1 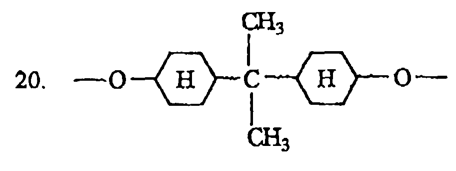 n = 1

5. The method according to p.2, includes preferably using oligomers, chosen from the following group:

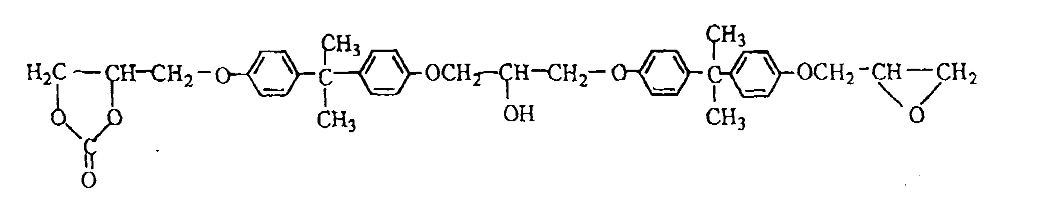

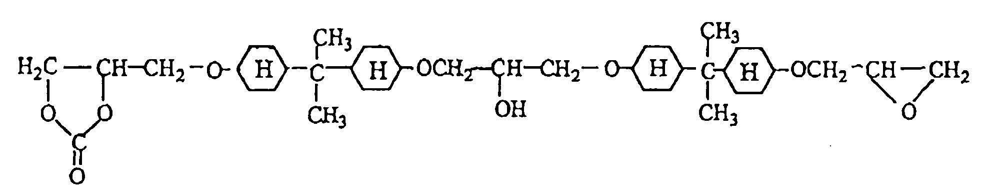

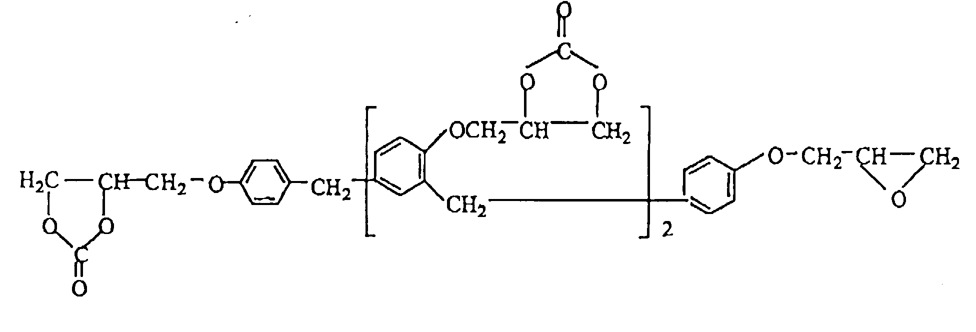

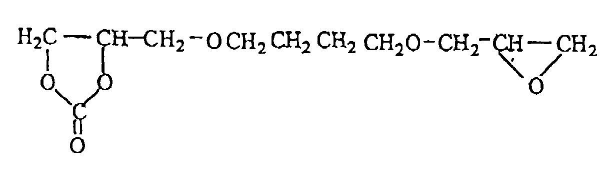

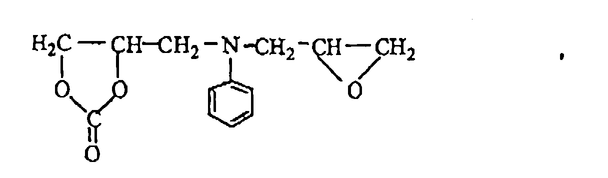

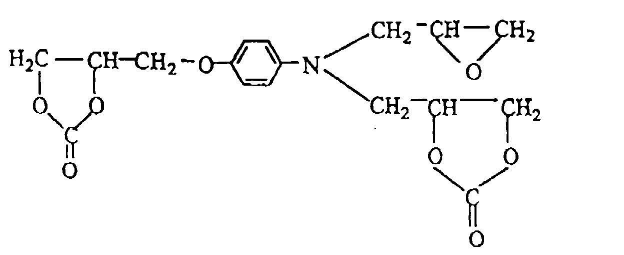

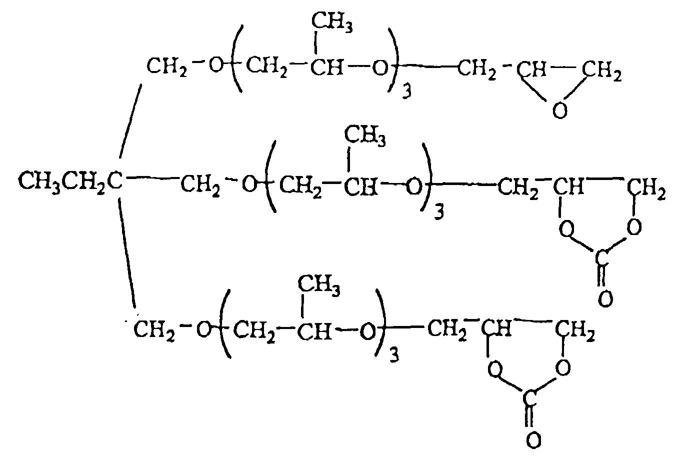

6. The method according to p.2, includes using aliphatic, cycloaliphatic diamines, chosen from the groups:
H₂N―R―NH₂
where R:
